# EUROPÄISCHE PATENTSCHRIFT

(11) **EP 2 926 841 B1**
(45) Veröffentlichungstag und Bekanntmachung des Hinweises auf die Patenterteilung: **02.05.2018**
(21) Anmeldenummer: 15159317.5
(22) Anmeldetag: 17.03.2015
(51) Int. Cl.: A61L 27/50, A01N 57/24, A61L 27/54, A61L 24/00, A61K 9/14, A61K 31/665

(54) **FOSFOMYCIN-ZUBEREITUNG ENTHALTENDES POLYMETHYLMETHACRYLAT-KNOCHENZEMENTPULVER**
POLYMETHYLMETHACRYLATE BONE CEMENT POWDER CONTAINING FOSFOMYCIN PREPARATION
POUDRE DE CIMENT OSSEUX EN POLYMÉTHYMÉTHACRYLATE CONTENANT UNE PRÉPARATION DE FOSFOMYCINE

(30) Priorität: 02.04.2014 DE 102014104676
(43) Veröffentlichungstag der Anmeldung: 07.10.2015
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A2- 1 949 918
- CN-A- 102 784 118

## Beschreibung

Gegenstand der Erfindung ist ein eine Fosfomycin-Zubereitung enthaltendes Polymethylmethacrylat-Knochenzementpulver.
Gelenkendoprothesen werden in großem Umfang bei unterschiedlichsten Gelenkerkrankungen mit sehr großem Erfolg zur Erhaltung der Bewegungsfunktionen der Patienten eingesetzt. Leider kommt es bei einem geringen Anteil der Patienten zu Infektionen an den Gelenkendoprothesen und dem umgebenden Knochen- und Weichgewebe. Sehr häufig werden zur Behandlung dieser Infektionen der einzeitige und auch der zweizeitige Gelenkendoprothesenwechsel durchgeführt.

Für die dauerhafte mechanische Fixierung der Revisions-Gelenkendoprothesen haben sich Revisions-Polymethylmethacrylat-Knochenzemente bewährt, die ein Antibiotikum oder zwei oder auch mehrere Antibiotika enthalten. Durch diese Antibiotika wird die Revisions-Gelenkendoprothese und das umgebende Knochen- und Weichgewebe zumindest unmittelbar postoperativ vor einer erneuten mikrobiellen Besiedlung geschützt. Neben der individuellen Beimischung von Antibiotika durch den Arzt haben sich industriell hergestellte Revisons-Polymethylmethacrylat-Knochenzemente bewährt.

So werden von der Heraeus Medical GmbH die Revisions-Polymethylmethacrylat-Knochenzemente Copal® G+C und Copal® G+V hergestellt und vertrieben. Copal® G+C enthält die Kombination Gentamicin und Clindamycin. Copal® G+V enthält die Antibiotika Gentamicin und Vancomycin. Die Kombination Gentamicin mit Vancomycin eignet sich bisher besonders dann, wenn die Infektion der Gelenkendoprothese durch Methicillin-resistente Staphylokokken (MRSA, MRSE) hervorgerufen wird.

Seit einigen Jahren sind jedoch auch Vancomycin-resistente Staphylokken- und Enterokokken-Stämme bekannt. Es ist damit zu rechnen, dass diese Vancomycin-resistenten Bakterien in den nächsten Jahren eine zunehmende Rolle als Verursacher von Gelenk-assoziierten Infektionen spielen werden. Daher ist es sinnvoll, einen Revisions-Polymethylmethacrylat-Knochenzement zu entwickeln, der mindestens ein Antibiotikum enthält, das wirksam gegenüber Vancomycin-resistenten Bakterien ist. Daneben spielen auch zunehmend problematische Gram-negative Bakterien eine Rolle als Verursacher von Gelenk-assoziierten Infektionen. Es handelt sich dabei insbesondere um sogenannte ESBL-Stämme.

Ein Antibiotikum mit einem sehr breiten Wirkungsspektrum stellt Fosfomycin dar. Das Antibiotikum Fosfomycin ((2*R*,3*S*)-3-Methyloxiranphosphonsäure, CAS 23155-02-4) wurde 1969 entdeckt (D. Hendlin et al.: Phosphonomycin a new antibiotic produced by strains of Streptomyces. Science 96 (1969) 122-123.)

Fosfomycin hemmt das bakterielle Enzym MurA (UDP-*N*-Acetylglucosaminenolpyruvyl-transferase) (F. M. Kahan et al.: The mechanism of action of fosfomycin (phosphonomycin). Ann N Y Acad Sci 235 (1974) 364-386.; E. D. Brown et al.: MurA (MurZ), the enzyme that catalyzes the first committed step in peptidoglycan biosynthesis, is essential in Escherichia coli. J. Bacteriol. 177 (14) (1995) 4194-4197.). Dieses katalysiert den ersten Schritt der Mureinbiosynthese. Bei diesem Schritt wird auf die Hydroxylgruppe an Position 3 des UDP-*N-*Acetylglucosamins eine Enolpyruvyleinheit ausgehend von Phosphoenolpyruvat (PEP) transferiert. Das bedeutet, es entsteht ein Milchsäureether an der Position 3 des UDP-*N*-Acetylglucosamins. Bei Störung dieses Schritts durch Fosfomycin wird die bakterielle Zellwandsynthese inhibiert.

Fosfomycin wirkt bei sensiblen Bakterien bakterizid. Fosfomycin erfasst Gram-negative und Gram-positive Bakterien, wobei auch Methicillin-resistente Staphylokokken eingeschlossen sind (W. Graninger et al.: In vitro activity of fosfomycin against methicillin-susceptible and methicillin-resistant Staphylococcus aureus. Infection 12 (1984) 293-295.). Fosfomycin ist auch bei Vancomycin-resistenten Staphylokokkus aureus (VRS) und auch bei Vancomycin-resistenten Enterokokken wirksam (F. Allerberger, I. Klare: In-vitro activity of fosfomycin against vancomycin-resistant enterococci. J Antimicrob Chemother 43 (1999) 211-217.; T. Hara et al.: Antimicrobial activity of fosfoymcin against beta-lactamaseproducing methicillin-sensitive Staphylococcus aureus and methicillin sensitive coagulase-negative staphylococci. Jpn J Antibiot 56 (20013) 142-147.). Daneben ist Fosfomycin auch bei ESBL wirksam (M. E. Falagas et al.: Fosfomycin for the treatment of multidrug-resistant, including extended-spectrum β-lactamase producing enterobacteriaceae infections: a systematic review. Lancet Infect Dis 10 (2010) 43-50.).

Für die pharmazeutische Verwendung wird Fosfomycin in Salze überführt, die hinreichend lagerstabil sind und deren wässrige Lösungen einen physiologisch tolerierbaren pH-Wert haben. In der europäischen Pharmakopoe werden drei Salze des Fosfomycins beschrieben. Es handelt sich dabei um das Monohydrat des Calciumsalzes des Fosfomycins (CAS 26016-98-8), um das Dinatriumsalz des Fosfomycins (CAS 26016-99-9) und um Trometamol-Fosfomycin (CAS 78964-85-9).

Das Natriumsalz und das Calciumsalz des Fosfomycins sind außerordentlich hygroskopisch. Beide Salze ziehen Luftfeuchtigkeit an und zerfließen dabei. Versuche zeigten, dass diese Salze im trockenen Zustand in Zementpulver von Polymethylmethacrylat-Knochenzementen integriert werden können. Aber diese Zementpulver ziehen bei Lagerung an Luft ebenfalls Luftfeuchtigkeit, wobei die Antibiotika-Partikel zerfließen. Dabei kann es zur Verklumpung des Zementpulvers kommen. Daher sind beide Salze des Fosfomycins für die industrielle Herstellung von antibiotischen Polymethylmethacrylat-Knochenzementpulvern nicht oder nur sehr bedingt geeignet. Das Trometamol-Fosfomycin ist auch etwas hygroskopisch, aber etwas weniger als das Calciumsalz und das Dinatriumsalz des Fosfomycins.

Das Calciumsalz des Fosfomycins ist für die Herstellung von antibiotischen Polymethylmethacrylat-Knochenzementen besonders interessant. Calcium-Ionen sind ein essentieller Bestandteil der mineralischen Knochenbestandteile. Daher kann erwartet werden, dass ein Polymethylmethacrylat-Knochenzement, der das Calciumsalz des Fosfomycins enthält, vom Knochengewebe toleriert wird. Bedingt durch die Hygroskopie des Calciumsalzes des Fosfomycins ist jedoch eine einfache Einbringung dieses Salzes in Polymethylmethacrylat-Zementpulver praktisch nicht möglich. Eine industrielle Herstellung von Polymethylmethacrylat-Knochenzementpulver ist praktisch nicht realisierbar. CN102784118 A offenbart eine Tablette hergestellt durch Gefriertrocknung einer Fosfomycin-Zubereitung folgender Zusammensetzung (in Gewichtsprozenten): 10-24% Fosfomycin Calcium, 24-50% Mannitol, 2-4% Gelatin, 32-74% Hydroxypropyl-beta-Cyclodextrin, 0.1-0.2% Trichlorsucrose und 50% Wasser.

EP1949918 A offenbart einen Revisions-PMMA-Knochenzement mit Pulver- und Flüssig-Komponente, wobei in der Pulverkomponente zwei oder mehr granuläre Antibiotika enthalten sind.

Aufgabe der Erfindung besteht darin, die vorstehend genannten Nachteile des Standes der Technik zu überwinden. Eine Aufgabe der Erfindung besteht darin, ein Polymethylmethacrylat-Knochenzementpulver zu entwickeln, der die Zubereitung des Calciumsalzes des Fosfomycins enthält und die mechanischen Mindestanforderungen der ISO 5833 für Polymethylmethacrylat-Knochenzemente erfüllt. Es sollte eine bei Raumtemperatur pulverförmige Zubereitung des Calciumsalzes des Fosfomycins bereitgestellt werden, die nur eine minimale Hygroskopie zeigt. Die Zubereitung soll bei Raumtemperatur und einer relativen Luftfeuchtigkeit von 50 % durch Anlagerung von Luftfeuchtigkeit nicht zerfließen. Weiterhin soll diese Zubereitung als rieselfähiger pulverförmiger Feststoff vorliegen, der problemlos gemahlen werden kann, ohne dass die Zubereitung Luftfeuchtigkeit anzieht und zerfließt.
Die Aufgabe der Erfindung wurde überraschenderweise durch einen Knochenzement gelöst, der eine Zubereitung von dem Calciumsalz des Fosfomycins mit einem Zuckeralkohol mit einem Schmelzpunkt größer 70°C enthält.
Die Erfindung betrifft daher ein Knochenzementpulver, umfassend eine Fosfoymcin-Zubereitung, wobei die Fosfomycin-Zubereitung
a) ein Calciumsalz des Fosfomycins und
b) mindestens einen Zuckeralkohol mit Schmelzpunkt größer 70 °C umfasst, und
das Gewichtsverhältnis von Zuckeralkohol zu Calciumsalz des Fosfomycins zum mindestens 0,8 beträgt.

Die Erfindung beruht auf der überraschenden Erkenntnis, das Gemische aus dem Calciumsalz des Fosfoymcins und mindestens einem Zuckeralkohol, der einen Schmelzpunkt größer 70 °C aufweist, im Gegensatz zu dem Calciumsalz des Fosfomycins deutlich weniger bzw. kaum hygroskopisch sind. Die Gemische können problemlos an der Luft mit eine relativen Luftfeuchtigkeit von 50 % bei Raumtemperatur gelagert werden, ohne dass die Zubereitung durch angelagerte Luftfeuchtigkeit zerfließt oder sich zu einer halbflüssigen Masse zusammenballt, wie es beim Calciumsalz des Fosfomycins der Fall ist. Die

Fosfomycin-Zubereitung kann problemlos aufgemahlen werden und das erhaltene Pulver zeigt keine ausgeprägte Hygroskopie und ist problemlos zu handhaben.

Ohne sich an eine Theorie binden zu wollen, wird vermutet, dass die überraschende Verbesserung des hygroskopischen Verhaltens darauf beruht, dass der Zuckeralkohol mit den Calciumionen wechselwirkt, wodurch die Tendenz der Calciumionen, Wasser anzuziehen, deutlich reduziert wird.

Mit der Fosfomycin-Zubereitung geling es, nicht klumpende frei rieselnde Polymethylmethacrylat-Knochenzementpulver in Gegenwart von nicht getrockneter Luft herzustellen. Dadurch kann mit technisch üblichen Geräten bzw. Maschinen ohne spezielle kostenintensive Schutzvorkehrungen gegenüber Luftfeuchtigkeit ein Calcium-Fosfomycin enthaltendes Zementpulver z.B. für Revisionszemente hergestellt werden.

Im folgenden wird die Erfindung ausführlich erläutert.

Wie bereits eingangs erwähnt, ist Fosfomycin (2*R*,3*S*)-3-Methyloxiranphosphonsäure, CAS 23155-02-4. Das Calciumsalz des Fosfomycins weist in der Regel Hydratwasser auf. Es wird auch als Monohydrat des Calciumsalzes des Fosfomycins (CAS 26016-98-8) oder Calcium-Fosfoymcin-Monohydrat bezeichnet. Das Calciumsalz von Fosfomycin ist im Handel erhältlich, z.B. von Ecros, Spanien. Das Calciumsalz von Fosfomycin ist ein hygroskopisches Pulver.

Als Zuckeralkohol eignet sich jeder beliebige Zuckeralkohol mit einem Schmelzpunkt größer 70°C. Zuckeralkohole werden auch als Alditole bezeichnet. Es können ein oder mehrere Zuckeralkohole eingesetzt werden. Als Zuckeralkohole können alle Stereoisomere oder beliebige Mischungen von Stereoisomeren eingesetzt werden. Zuckeralkohole sind toxikologisch unbedenklich und kostengünstig.

Der für die Fosfomycin-Zubereitung eingesetzte Zuckeralkohol ist bevorzugt ein Zuckeralkohol mit 4, 5 oder 6 Kohlenstoffatomen oder eine Mischung davon. Geeignete Beispiele für einen Zuckeralkohol sind Erythritol, Threitol, Xylitol Mannitol und Sorbitol, wobei Erythritol, Xylitol und Mannitol besonders bevorzugt sind.

Das Gewichtsverhältnis (b/a) von Zuckeralkohol (b) zu Calciumsalz des Fosfomycins (a) in der Fosfomycin-Zubereitung beträgt mindestens 0,8, bevorzugt mindestens 1 und besonders bevorzugt mindestens 1,5. Das Gewichtsverhältnis von Zuckeralkohol zu Calciumsalz des Fosfomycins in der Fosfomycin-Zubereitung beträgt in der Regel nicht mehr als 5, bevorzugt nicht mehr als 4 und besonders bevorzugt nicht mehr als 3,5. Es sind aber auch höhere Gewichtsanteile an Zuckeralkohol prinzipiell möglich. Auch in diesem Fall werden nicht hygroskopische Pulver erhalten. Allerdings erhöht sich dadurch unnötigerweise die Menge an nicht wirksamem Material (Zuckeralkohol) im Zement, was gewöhnlich nicht zweckmäßig ist, da die mechanische Stabilität geschwächt wird.

Das Gewichtsverhältnis von Zuckeralkohol zu Calciumsalz des Fosfomycins liegt bevorzugt im Bereich von 0,8 bis 5, bevorzugter von 1 bis 4 und besonders bevorzugt von 1,5 bis 3,5, wobei das Gewichtsverhältnis von Zuckeralkohol zu Calciumsalz des Fosfomycins besonders bevorzugt im Bereich von 2 bis 3 liegt, z.B. etwa 2 zu 1 oder etwa 3 zu 1.

Die Fosfomycin-Zubereitung ist insbesondere eine feste Zubereitung, d.h. ein Feststoff, und liegt vorzugsweise als Pulver vor. Gegebenenfalls kann es auch in Form eines Granulats vorliegen. Die Fosfomycin-Zubereitung ist bevorzugt eine erstarrte Schmelze von Zuckeralkohol mit darin dispergiertem Calciumsalz des Fosfomycins, wobei die erstarrte Schmelze vorzugsweise in gemahlener bzw. pulverisierter Form vorliegt. Sie kann gegebenenfalls auch in granulierter Form eingesetzt werden. In der Regel ist die Fosfomycin-Zubereitung ein farbloser, nichthygrospkopischer pulverförmiger Feststoff.

Die Fosfomycin-Zubereitung kann gegebenenfalls ein oder mehrere Zusatzstoffe enthalten. Es ist aber bevorzugt, dass die Fosfomycin-Zubereitung aus dem Calciumsalz des Fosfomycins und dem oder den Zuckeralkoholen besteht oder im wesentlichen besteht.

Die Zubereitung wird vorzugsweise in einfacher Weise durch Suspendieren des Calciumsalzes des Fosfomycins in einer Schmelze des mindestens einen Zuckeralkohols, Abkühlen und Aufmahlen der erstarrten Schmelze hergestellt. Das Verfahren zur Herstellung der Fosfomycin-Zubereitung umfasst folgende Schritte:
a) der mindestens eine Zuckeralkohol wird aufgeschmolzen,
b) in der Schmelze des Zuckeralkohols wird das Calciumsalz des Fosfomycins unter Rühren suspendiert,
c) die Schmelze wird nach der Suspendierung des Calciumsalzes des Fosfomycins abgekühlt, um eine erstarrte Schmelze mit darin dispergiertem Calciumsalz des Fosfomycins zu erhalten, und
d) die erstarrte Schmelze wird aufgemahlen, um einen pulverförmigen Feststoff zu erhalten.

In Schritt b) wird das Calciumsalz des Fosfomycins insbesondere als Feststoff, bevorzugt als Pulver, in die Schmelze des Zuckeralkohols unter Rühren gegeben. In Schritt c) wird die Schmelze vorzugsweise unmittelbar nach der Suspendierung abgekühlt, um eine erstarrte Schmelze mit darin dispergiertem Calciumsalz des Fosfomycins zu erhalten. In der Regel erfolgt die Abkühlung auf Umgebungstemperatur bzw. Raumtemperatur. Es ist bevorzugt, dass Abkühlung der Schmelze rasch erfolgt. Zur Abschreckung können die dem Fachmann bekannten üblichen Maßnahmen durchgeführt werden, z.B. eine externe Kühlung mit Wasser oder Eiswasser.

Die erstarrte Schmelze von Zuckeralkohol mit darin dispergiertem Calciumsalz des Fosfomycins lässt sich in Schritt d) leicht aufmahlen, z.B. manuell mit einem Mörser oder in einer Mühle. Durch das Mahlen fällt ein farbloser, nichthygroskopischer, pulverförmiger Feststoff an.

Die Fosfomycin-Zubereitung eignet sich für den Einsatz als antibiotisches Mittel für pulverförmige Zusammensetzungen, insbesondere für Polymethylmethacrylat-Knochenzementpulver. Dadurch können die Probleme mit den hygroskopischen Eigenschaften des Calciumsalzes von Fosfomycin vermieden werden.

Polymethylmethacrylat-Knochenzemente liegen in der Regel als Zweikomponentensysteme vor. Die erste Komponente ist gewöhnlich ein Knochenzementpulver. Das Knochenzementpulver wird auch als Polymethylmethacrylat-Knochenzementpulver bezeichnet. Die zweite Komponente enthält ein polymerisierbares Monomer, in der Regel Methylmethacrylat, und ist üblicherweise eine Flüssigkeit. Durch die Mischung der beiden Komponenten wird ein plastisch verformbarer Knochenzementteig erhalten, der nach einer gewissen Zeit aushärtet.

Die Fosfomycin-Zubereitung eignet sich insbesondere als antibiotisches Mittel für ein Knochenzementpulver für einen Polymethylmethacrylat-Knochenzement. Die Erfindung betrifft daher ein Knochenzementpulver, das die erfindungsgemäße Fosfomycin-Zubereitung wie vorstehend beschrieben, vorzugsweise als Pulver, umfasst.

Das Knochenzementpulver, das die Fosfomycin-Zubereitung umfasst, enthält ferner bevorzugt wenigstens ein partikuläres Polymethylmethacrylat oder Polymethylmethacrylat-co-polymer, einen partikulären Röntgenopaker, einen Radikalstarter und gegebenenfalls ein weiteres Antibiotikum und/oder Antiseptikum. Es ist bevorzugt, dass, bezogen auf das Gesamtgewicht des Knochenzementpulvers, die Fosfomycin-Zubereitung in einem Anteil von 1 bis 20 Gewichtsprozent in dem Knochenzementpulver enthalten ist.

Bei dem partikulären Polymethylmethacrylat oder Polymethylmethacrylat-co-polymer handelt es sich insbesondere um unvernetztes Polymer bzw. Copolymer. Das partikuläre Polymethylmethacrylat oder Polymethylmethacrylat-co-polymer ist bevorzugt ein Suspensionspolymerisat.

Als partikulären Röntgenopaker können alle üblichen partikulären Röntgenopaker eingesetzt werden. Beispiele sind Metalloxide, insbesondere Zirkoniumdioxid, Bariumsulfat, toxikologisch unbedenkliche Schwermetallpartikel, z.B. Tantal, Ferrit und Magnetit, oder toxikologisch unbedenkliche Calciumsalze.

Der Radikalstarter dient nach der Mischung mit der zweiten Komponente zur Initiierung der Polymerisation des in der zweiten Komponente enthaltenen Monomers, in der Regel Methylmethacrylat. Es können alle üblichen Radikalstarter verwendet werden. Beispiele sind Peroxide und Azoverbindungen, wobei Peroxide und insbesondere Dibenzoylperoxid bevorzugt sind.

Das Knochenzementpulver kann gegebenenfalls ein oder mehrere weitere Antibiotika und/oder ein oder mehrere Antiseptika enthalten. Beispiele für weitere Antibiotika, die gegebenenfalls im Knochenzementpulver enthalten sein können, sind Gentamicin, Tobramycin, Clindamycin, Vancomycin, Teicoplanin und Daptomycin. Beispiele für Antiseptika, die gegebenenfalls im Knochenzementpulver enthalten sein können, sind Octenidindihydrochlorid, Polyhexanid, Calciumperoxid und Harnstoffperoxid.

Die mit dem Knochenzementpulver zu vermischende zweite Komponente zur Herstellung eines Zementteigs kann z.B. eine auf diesem Gebiet übliche Monomerflüssigkeit sein. Eine solche Monomerflüssigkeit umfasst z.B. Methylmethacrylat und ein tertiäres Amin, wie N,N-Dimethyl-p-toluidin, sowie gegebenenfalls einen Stabilisator, wie p-Hydrochinon, und/oder einen Farbstoff.

Mit dem erfindungsgemäßen Knochenzementpulver kann durch Vermischung mit üblicher Monomerflüssigkeit, die z.B. aus Methylmethacrylat, N,N-Dimethyl-p-toluidin, p-Hydrochinon zusammengesetzt ist, ein plastisch verformbarer Knochenzementteig hergestellt werden, der nach Aushärtung die Anforderungen der ISO 5833 hinsichtlich der Biegefestigkeit von mindestens 50 MPa, des Biegemoduls von mindestens 1800 MPa und der Druckfestigkeit von mindestens 70 MPa erfüllt.

Das Knochenzementpulver, das die Fosfomycin-Zubereitung enthält, eignet sich insbesondere zur Herstellung von Revisions-Polymethylmethacrylat-Knochenzementen, zur Herstellung von Spacern und zur Herstellung von lokalen Wirkstofffreisetzungssystemen.

Unter Revisions-Polymethylmethacrylat-Knochenzementen versteht man Polymethylmethacrylat-Knochenzemente, die zur dauerhaften Fixierung von Revisionsgelenkendoprothesen bestimmt sind, die im Rahmen der einzeitigen und auch der zweizeitigen septischen Revision von infizierten Gelenkendoprothesen verwendet werden. Unter dem Begriff Spacer werden temporäre Implantate verstanden, die im Rahmen der zweizeitigen septischen Revision von infizierten Gelenkendoprothesen als temporäre Platzhalter eingesetzt werden. Das Polymethylmethacrylat-Knochenzementpulver kann auch zur Herstellung von lokalen Wirkstofffreisetzungssystemen eingesetzt werden, wobei das Zementpulver mit üblichen Gemischen aus Methylmethacrylat und einem tertiären Amin, z.B. N,N-Dimethyl-p-toluidin, vermischt wird, wobei ein selbsthärtender Zementteig entsteht, der in beliebige Form gegossen oder modelliert werden kann, wobei nach der Aushärtung durch radikalische Polymerisation mechanisch stabile Formkörper entstehen. Diese können im Rahmen der lokalen Antibiotika-Therapie verwendet werden. Die Wirkstofffreisetzungssysteme können kugelförmig, bohnenförmig, stabförmig ausgebildet sein. Es ist auch möglich, kugelförmige oder bohnenförmige Formkörper auf biokompatible Drähte aufzubringen.

Die Erfindung wird durch nachstehende Beispiele weiter erläutert, die die Erfindung aber in keiner Weise beschränken sollen.

### Beispiele

Als Calciumsalz des Fosfomycins wird in den folgenden Beispielen ein Monohydrat des Calciumsalzes des Fosfomycins eingesetzt, welches von der Firma Ecros, Spanien, bezogen wurde und nachfolgend vereinfacht als Calcium-Fosfoymcin-Monohydrat bezeichnet wird.

### Beispiel 1

Es wurden 4,00 g Erythritol (Schmelzpunkt 120 °C, Sigma-Aldrich) in einem Porzellantiegel eingewogen. Dann wurde der Porzellantiegel auf eine Heizplatte gestellt, die eine Temperatur von 150 °C hatte. Das Erythritol bildete eine klare Schmelze. Zu dieser Schmelze wurden 4,00 g Calcium-Fosfoymcin-Monohydrat gegeben. Unter intensivem Rühren wurde das Calcium-Fosfoymcin-Monohydrat mit der Schmelze vermischt. Danach wurde das Gemisch sofort auf Raumtemperatur gekühlt. Die Schmelze erstarrte als farbloser Feststoff. Dieser wurde aus dem Porzellantiegel entnommen und anschließend gemörsert. Es entstand ein farbloser, pulverförmiger Feststoff, der problemlos an der Luft gelagert werden konnte, ohne dass die Zubereitung verklumpte oder unter Anlagerung von Luftfeuchtigkeit zerfloss.

### Beispiel 2

Es wurden 8,00 g Erythritol (Schmelzpunkt 120 °C, Sigma-Aldrich) in einem Porzellantiegel eingewogen. Dann wurde der Porzellantiegel auf eine Heizplatte gestellt, die eine Temperatur von 150 °C hatte. Das Erythritol bildete eine klare Schmelze. Zu dieser Schmelze wurden 4,00 g Calcium-Fosfoymcin-Monohydrat gegeben. Unter intensivem Rühren wurde das Calcium-Fosfoymcin-Monohydrat mit der Schmelze vermischt. Danach wurde das Gemisch sofort auf Raumtemperatur gekühlt. Die Schmelze erstarrte als farbloser Feststoff. Dieser wurde aus dem Porzellantiegel entnommen und anschließend gemörsert. Es entstand ein farbloser, pulverförmiger Feststoff, der problemlos an der Luft gelagert werden konnte, ohne dass die Zubereitung verklumpte oder unter Anlagerung von Luftfeuchtigkeit zerfloss.

### Beispiel 3

Es wurden 12,00 g Erythritol (Schmelzpunkt 120 °C, Sigma-Aldrich) in einem Porzellantiegel eingewogen. Dann wurde der Porzellantiegel auf eine Heizplatte gestellt, die eine Temperatur von 150 °C hatte. Das Erythritol bildete eine klare

Schmelze. Zu dieser Schmelze wurden 4,00 g Calcium-Fosfoymcin-Monohydrat gegeben. Unter intensivem Rühren wurde das Calcium-Fosfoymcin-Monohydrat mit der Schmelze vermischt. Danach wurde das Gemisch sofort auf Raumtemperatur gekühlt. Die Schmelze erstarrte als farbloser Feststoff. Dieser wurde aus dem Porzellantiegel entnommen und anschließend gemörsert. Es entstand ein farbloser, pulverförmiger Feststoff, der problemlos an der Luft gelagert werden konnte, ohne dass die Zubereitung verklumpte oder unter Anlagerung von Luftfeuchtigkeit zerfloss.

### Beispiel 4

Es wurden 8,00 g Xylitol (Schmelzpunkt 94 °C, Sigma-Aldrich) in einem Porzellantiegel eingewogen. Dann wurde der Porzellantiegel auf eine Heizplatte gestellt, die eine Temperatur von 150 °C hatte. Das Xylitol schmolz auf und bildete eine klare Schmelze. Zu dieser Schmelze wurden 4,00 g Calcium-Fosfoymcin-Monohydrat gegeben. Unter intensivem Rühren wurde das Calcium-Fosfoymcin-Monohydrat mit der Schmelze vermischt. Danach wurde das Gemisch sofort auf Raumtemperatur gekühlt. Die Schmelze erstarrte als farbloser Feststoff. Dieser wurde aus dem Porzellantiegel entnommen und anschließend gemörsert. Es entstand ein farbloser, pulverförmiger Feststoff, der problemlos an der Luft gelagert werden konnte, ohne dass die Zubereitung verklumpte oder unter Anlagerung von Luftfeuchtigkeit zerfloss.

### Beispiel 5

Es wurden 12,0 g Xylitol (Schmelzpunkt 94 °C, Sigma-Aldrich) in einem Porzellantiegel eingewogen. Dann wurde der Porzellantiegel auf eine Heizplatte gestellt, die eine Temperatur von 150 °C hatte. Das Xylitol bildete eine klare Schmelze. Zu dieser Schmelze wurden 4,00 g Calcium-Fosfoymcin-Monohydrat gegeben. Unter intensivem Rühren wurde das Calcium-Fosfoymcin-Monohydrat mit der Schmelze vermischt. Danach wurde das Gemisch sofort auf Raumtemperatur gekühlt. Die Schmelze erstarrte als farbloser Feststoff. Dieser wurde aus dem Porzellantiegel entnommen und anschließend gemörsert. Es entstand ein farbloser, pulverförmiger Feststoff, der problemlos an der Luft gelagert werden konnte, ohne dass die Zubereitung verklumpte oder unter Anlagerung von Luftfeuchtigkeit zerfloss.

### Beispiel 6

Es wurden 8,00 g Mannitol (Schmelzpunkt 166 °C, Sigma-Aldrich) in einem Porzellantiegel eingewogen. Dann wurde der Porzellantiegel auf eine Heizplatte gestellt, die eine Temperatur von 230 °C hatte. Das Erythritol bildete eine klare Schmelze. Zu dieser Schmelze wurden 4,00 g Calcium-Fosfoymcin-Monohydrat gegeben. Unter intensivem Rühren wurde das Calcium-Fosfoymcin-Monohydrat mit der Schmelze vermischt. Danach wurde das Gemisch sofort auf Raumtemperatur gekühlt. Die Schmelze erstarrte als farbloser Feststoff. Dieser wurde aus dem Porzellantiegel entnommen und anschließend gemörsert. Es entstand ein farbloser, pulverförmiger Feststoff, der problemlos an der Luft gelagert werden konnte, ohne dass die Zubereitung verklumpte oder unter Anlagerung von Luftfeuchtigkeit zerfloss.

### Polymethylmethacrylat-Knochenzementpulver für die Beispiele 7 bis 12

Es wurde jeweils ein Gemisch aus 88,53 g partikuläres Polymethylmethacrylat-comethylacrylat (Suspensionspolymerisat), 10,00 g Zirkoniumdioxid und 1,47 g 75 %iges Dibenzoylperoxid (Dibenzoylperoxid phlegmatisiert durch 25 Gewichtsprozent Wasser) durch Vermahlung mit Hilfe eines Turbula-Mischers hergestellt.

### Beispiel 7

40,0 g des Polymethylmethacrylat-Knochenzementpulvers wurden mit 4,00 g der Fosfomycin-Zubereitung des Beispiels 1 vermischt. 44,0 g dieses Zementpulvers wurden mit 20 ml Palacos-Monomerflüssigkeit (Gemisch aus Methylmethacrylat, Chlorophyllin E141, N,N-Dimethyl-p-toluidin und p-Hydrochinon) vermischt. Es wurde 30 Sekunden gerührt. Es entstand ein plastisch verformbarer Zementteig. Mit diesem wurden streifenförmige Probekörper der Abmessungen 75 mm x 10 mm x 3,3 mm für die Prüfung der Biegefestigkeit und des Biegemoduls gemäß ISO 5833 hergestellt. Weiterhin wurden zylinderförmige Probekörper (Durchmesser 6 mm, Höhe 12 mm) für die Prüfung der Druckfestigkeit hergestellt.

### Beispiel 8

40,00 g des Polymethylmethacrylat-Knochenzementpulvers wurden mit 4,00 g der Fosfomycin-Zubereitung des Beispiels 2 vermischt. 44,0 g dieses Zementpulvers wurden mit 20 ml Palacos-Monomerflüssigkeit vermischt. Es wurde 30 Sekunden gerührt. Es entstand ein plastisch verformbarer Zementteig. Es wurden dann wie in Beispiel 7 Prüfkörper hergestellt.

### Beispiel 9

40,00 g des Polymethylmethacrylat-Knochenzementpulvers wurden mit 4,00 g der Fosfomycin-Zubereitung des Beispiels 3 vermischt. 44,0 g dieses Zementpulvers wurden mit 20 ml Palacos-Monomerflüssigkeit vermischt. Es wurde 30 Sekunden gerührt. Es entstand ein plastisch verformbarer Zementteig. Es wurden dann wie in Beispiel 7 Prüfkörper hergestellt.

### Beispiel 10

40,00 g des Polymethylmethacrylat-Knochenzementpulvers wurden mit 4,00 g der Fosfomycin-Zubereitung des Beispiels 4 vermischt. 44,0 g dieses Zementpulvers wurden mit 20 ml Palacos-Monomerflüssigkeit vermischt. Es wurde 30 Sekunden gerührt. Es entstand ein plastisch verformbarer Zementteig. Es wurden dann wie in Beispiel 7 Prüfkörper hergestellt.

### Beispiel 11

40,00 g des Polymethylmethacrylat-Knochenzementpulvers wurde mit 4,00 g der Fosfomycin-Zubereitung des Beispiels 5 vermischt. 44,0 g dieses Zementpulvers wurden mit 20 ml Palacos-Monomerflüssigkeit vermischt. Es wurde 30 Sekunden gerührt. Es entstand ein plastisch verformbarer Zementteig. Es wurden dann wie in Beispiel 7 Prüfkörper hergestellt.

### Beispiel 12

40,00 g des Polymethylmethacrylat-Knochenzementpulvers wurden mit 4,00 g der Fosfomycin-Zubereitung des Beispiels 6 vermischt. 44,0 g dieses Zementpulvers wurden mit 20 ml Palacos-Monomerflüssigkeit vermischt. Es wurde 30 Sekunden gerührt. Es entstand ein plastisch verformbarer Zementteig. Es wurden dann wie in Beispiel 7 Prüfkörper hergestellt.

### Prüfung der mechanischen Parameter gemäß ISO 5833 für die Beispiele 7 bis 12

Die ISO 5833 fordert eine Biegefestigkeit von ≥ 50 MPa, ein Biegemodul von ≥ 1800 MPa und eine Biegefestigkeit von ≥ 70 MPa. Nach Lagerung der Probekörper der Beispiele 7 bis 12 bei 23 °C und einer relativen Luftfeuchtigkeit von 50 % über einen Zeitraum von 24 Stunden wurden die Biegefestigkeit, das Biegemodul und die Druckfestigkeit gemäß ISO 5833 bestimmt. Die Ergebnisse zeigen, dass die mechanischen Anforderungen der ISO5833 bezüglich der Biegefestigkeit, des Biegemoduls und der Druckfestigkeit von den Zementen der Beispiele 8-12 erfüllt wurden. Der Zement vom Beispiel 7 erfüllt die Anforderungen an das Biegemodul und die Druckfestigkeit. Jedoch die Biegefestigkeit befindet sich an der Grenze der Vorgabe der ISO 5833.

| Beispiel | Biegefestigkeit [MPa] | Biegemodul [MPa] | Druckfestigkeit [MPa] |
|---|---|---|---|
| 7 | 49,7 ± 2,7 | 3326 ± 61 | 97,5 ± 1,7 |
| 8 | 59,2 ± 3,4 | 3063 ± 140 | 98,5 ± 3,5 |
| 9 | 62,2 ± 2,8 | 3092 ± 68 | 99,5 ± 1,7 |
| 10 | 61,5 ± 2,0 | 2854 ± 50 | 96,8 ± 2,0 |
| 11 | 66,9 ± 2,7 | 3246 ± 120 | 99,4 ± 0,7 |
| 12 | 56,0 ± 1,7 | 3446 ± 86 | 98,6 ± 1,8 |

## Patentansprüche

1. Knochenzementpulver, umfassend eine Fosfoymcin-Zubereitung, wobei die Fosfomycin-Zubereitung
a) ein Calciumsalz des Fosfomycins und
b) mindestens einen Zuckeralkohol mit einem Schmelzpunkt größer 70 °C, umfasst, und
das Gewichtsverhältnis von Zuckeralkohol zu Calciumsalz des Fosfomycins mindestens 0,8 beträgt.

2. Knochenzementpulver nach Anspruch 1, **dadurch gekennzeichnet, dass** der Zuckeralkohol 4, 5 oder 6 Kohlenstoffatome aufweist, wobei der Zuckeralkohol bevorzugt aus Erythritol, Xylitol und Mannitol ausgewählt ist.

3. Knochenzementpulver nach Anspruch 1 oder Anspruch 2, **dadurch gekennzeichnet, dass** das Gewichtsverhältnis von Zuckeralkohol zu Calciumsalz des Fosfomycins mindestens 1 beträgt.

4. Knochenzementpulver nach irgendeinem der Ansprüche 1 bis 3, **dadurch gekennzeichnet, dass** die Fosfomycin-Zubereitung eine feste Zubereitung ist, vorzugsweise ein Pulver.

5. Knochenzementpulver nach irgendeinem der Ansprüche 1 bis 4, **dadurch gekennzeichnet, dass** die Fosfomycin-Zubereitung eine erstarrte Schmelze von Zuckeralkohol mit darin dispergiertem Calciumsalz des Fosfomycins ist.

6. Knochenzementpulver nach irgendeinem der Ansprüche 1 bis 5, **dadurch gekennzeichnet, dass** das Gewichtsverhältnis von Zuckeralkohol zu Calciumsalz des Fosfomycins im Bereich von 0,8 bis 5, bevorzugt von 1 bis 4 und bevorzugter von 1,5 bis 3,5 liegt.

7. Knochenzementpulver nach einem der vorhergehenden Ansprüche, ferner umfassend mindestens ein partikuläres Polymethylmethacrylat oder Polymethylmethacrylat-co-polymer, einen partikulären Röntgenopaker, einen Radikalstarter, wie Dibenzoylperoxid, und gegebenenfalls ein weiteres Antibiotikum und/oder Antiseptikum.

8. Knochenzementpulver nach einem der vorhergehenden Ansprüche, **dadurch gekennzeichnet, dass** die Fosfomycin-Zubereitung in einem Anteil von 1 bis 20 Gewichtsprozent im Knochenzementpulver enthalten ist.

9. Verwendung eines Knochenzementpulvers nach einem der einem der vorhergehenden Ansprüche zur Herstellung von Revisions-Polymethylmethacrylat-Knochenzementen, zur Herstellung von Spacern und zur Herstellung von lokalen Wirkstofffreisetzungssystemen.

## Claims

1. Bone cement powder, comprising a fosfomycin preparation, whereby the fosfomycin preparation comprises
a) a calcium salt of fosfomycin and
b) at least one sugar alcohol having a melting point above 70°C, and
the weight ratio of sugar alcohol to calcium salt of fosfomycin is at least 0.8.

2. Bone cement powder according to claim 1, **characterised in that** the sugar alcohol comprises 4, 5 or 6 carbon atoms, whereby the sugar alcohol preferably is selected from a erythritol, xylitol, and mannitol.

3. Bone cement powder according to claims 1 or 2, **characterised in that** the weight ratio of sugar alcohol to calcium salt of fosfomycin is at least 1.

4. Bone cement powder according to any one of the claims 1 to 3, **characterised in that** the fosfomycin preparation is a solid preparation, preferably a powder.

5. Bone cement powder according to any one of the claims 1 to 4, **characterised in that** the fosfomycin preparation is a solidified melt of sugar alcohol with calcium salt of fosfomycin dispersed in it.

6. Bone cement powder according to any one of the claims 1 to 5, **characterised in that** the weight ratio of sugar alcohol to calcium salt of fosfomycin is in the range of 0.8 to 5, preferably of 1 to 4, and more preferably of 1.5 to 3.5.

7. Bone cement powder according to any one of the preceding claims, further comprising at least one particulate polymethylmethacrylate or poly-methylmethacrylate-co-polymer, a particulate radiopaquer, a radical starter, such as dibenzoyl peroxide, and, if applicable, a further antibiotic and/or antiseptic agent.

8. Bone cement powder according to one of the preceding claims, **characterised in that** the fosfomycin preparation is present in the bone cement powder at a fraction of 1 to 20% by weight.

9. Use of a bone cement powder according to any one of the preceding claims for the production of revision polymethylmethacrylate bone cements, for the production of spacers, and for the production of local agent release systems.

## Revendications

1. Poudre de ciment osseux comprenant une préparation de fosfomycine, dans laquelle la préparation de fosfomycine comprend
a) un sel calcique de la fosfomycine et
b) au moins un polysaccharide avec un point de fusion supérieur à 70°C, et
le rapport pondéral du polysaccharide sur le sel calcique de la fosfomycine vaut au moins 0,8.

2. Poudre de ciment osseux selon la revendication 1, **caractérisée en ce que** le polysaccharide présente 4, 5 ou 6 atomes de carbone, dans laquelle le polysaccharide est de préférence sélectionné parmi l'érythritol, le xylitol et le mannitol.

3. Poudre de ciment osseux selon la revendication 1 ou la revendication 2, **caractérisée en ce que** le rapport pondéral du polysaccharide sur le sel calcique de la fosfomycine vaut au moins 1.

4. Poudre de ciment osseux selon l'une quelconque des revendications 1 à 3, **caractérisée en ce que** la préparation de fosfomycine est une préparation solide, de préférence une poudre.

5. Poudre de ciment osseux selon l'une quelconque des revendications 1 à 4, **caractérisée en ce que** la préparation de fosfomycine est une masse fondue solidifiée de polysaccharide avec du sel calcique de la fosfomycine dispersé en son sein.

6. Poudre de ciment osseux selon l'une quelconque des revendications 1 à 5, **caractérisée en ce que** le rapport pondéral du polysaccharide sur le sel calcique de la fosfomycine se situe dans la plage de 0,8 à 5, de préférence de 1 à 4 et de manière davantage préférée de 1,5 à 3,5.

7. Poudre de ciment osseux selon une des revendications précédentes, comprenant en outre au moins un polyméthylméthacrylate ou polyméthylméthacrylate-co-polymère particulaire, un opacifiant radiographique particulaire, un amorceur radicalaire, comme le peroxyde de dibenzoyle, et éventuellement un autre antibiotique et/ou antiseptique.

8. Poudre de ciment osseux selon une des revendications précédentes, **caractérisée en ce que** la préparation de fosfomycine est contenue dans une proportion de 1 à 20 pourcent en poids dans la poudre de ciment osseux.

9. Utilisation d'une poudre de ciment osseux selon une des revendications précédentes pour la fabrication de ciments osseux de polyméthylméthacrylate de révision, pour la fabrication d'écarteurs et pour la fabrication de systèmes de libération d'ingrédient actif locaux.
